# EUROPÄISCHE PATENTSCHRIFT

(11) **EP 2 742 028 B1**
(45) Veröffentlichungstag und Bekanntmachung des Hinweises auf die Patenterteilung: **01.04.2015**
(21) Anmeldenummer: 12743750.7
(22) Anmeldetag: 08.08.2012
(51) Int. Cl.: C07D 233/54

(54) **VERFAHREN ZUR REINIGUNG VON IONISCHEN FLÜSSIGKEITEN**
PROCESS FOR THE PURIFICATION OF IONIC LIQUIDS
PROCÉDÉ D'ÉPURATION DE LIQUIDES IONIQUES

(30) Priorität: 09.08.2011 EP 11176884
(43) Veröffentlichungstag der Anmeldung: 18.06.2014
(73) Patentinhaber: BASF SE, 67056 Ludwigshafen (DE)
(72) Erfinder: SIEMER, Michael, 68159 Mannheim (DE); KLEIN, Michael, 66879 Reichenbach-Steegen (DE); SON, Sunghee, 68159 Mannheim (DE); MASSONNE, Klemens, 67098 Bad Dürkheim (DE); VAGT, Uwe, 68165 Mannheim (DE)
(86) Internationale Anmeldenummer: PCT/EP2012/065542
(87) Internationale Veröffentlichungsnummer: WO 2013/021015

(56) Entgegenhaltungen:
- EP-A1- 1 470 846
- WO-A2-2008/140496

## Beschreibung

Die vorliegende Anmeldung schließt durch Verweis die am 09. August 2011 einreichte vorläufige US-Anmeldung 61/521384 ein.

Die Erfindung betrifft ein Verfahren zur Reinigung von Salzen K⁺X⁻, wobei K⁺ für ein beliebiges Kation und X⁻ für ein organisches Anion mit mindestens 6 C-Atomen steht, welches dadurch gekennzeichnet ist, dass
a) die Salze K⁺X⁻ in Zusammensetzungen vorliegen, die als Verunreinigungen wasserlösliche Salze (K⁺)ₙ Yⁿ⁻ enthalten, wobei K⁺ für das obige Kation, Yⁿ⁻ für ein anorganisches Anion oder ein von X⁻ verschiedenes organisches Anion mit maximal 10 C-Atomen und n für 1, 2 oder 3 steht,
b) die Anionen X- der Salze K⁺X⁻ durch Zusatz einer wasserlöslichen Wasserstoffsäure (H⁺)ₘ Z^{m-}, wobei m für 1, 2 oder 3 steht, in die Säuren HX überführt werden, wobei sich zwei Phasen ausbilden, eine organische Phase, welche HX enthält, und eine wässrige Phase, welche die wasserlösliche Salze (K⁺)ₘZ^{m-} und (K⁺)ₙYⁿ⁻ oder Säuren (H⁺)ₙ Yⁿ⁻ enthält,
c) die organische Phase abgetrennt wird,
d) die wässrige Phase über einen stark basischen Anionentauscher geführt wird, welcher die Anionen Z^{m-} und Yⁿ⁻ bindet und OH-frei setzt, so dass ein wässriges Gemisch, enthaltend K⁺ OH⁻ erhalten wird und
e) die organische Phase (enthaltend HX) und das erhaltene wässrige Gemisch (enthaltend KOH), gegebenenfalls unter Zusatz von weiterem HX, zu K⁺X⁻ und H₂O umgesetzt werden.

Salze mit einem organischen Kation sind z.B. als ionische Flüssigkeiten von Bedeutung. Ionische Flüssigkeiten haben einen Schmelzpunkt kleiner 200°C, insbesondere kleiner 100°C.

Für ionische Flüssigkeiten gibt es eine große Anzahl von technischen Verwendungen, z.B. als Lösemittel. Bei der Verwendung werden ionische Flüssigkeiten im Allgemeinen nicht verbraucht, sondern nur verunreinigt. Da es sich um hochpreisige Salze handelt, besteht ein Bedarf an besonders effektiven und günstigen Verfahren zur Aufarbeitung der bei der Verwendung erhaltenen Gemische, so dass eine Wiederverwendung erfolgen kann.

Bei der Verwendung von ionischen Flüssigkeiten zur Auflösung von Zellulose entstehen z.B. Gemische, welche neben der ionischen Flüssigkeit Lösemittel, insbesondere Wasser, eingebrachte Verunreinigungen, z.B. eingebrachte Säuren, Salze oder Abbauprodukte der Zellulose und der ionischen Flüssigkeit enthalten. Abbauprodukte der Zellulose sind insbesondere kurzkettige Carbonsäuren, welche dann auch durch Gleichgewichtsreaktionen als zum Kation der ionischen Flüssigkeit zugehörige Anionen vorliegen. Auch anorganische Kationen können als Verunreinigungen vorliegen. Unerwünscht ist insbesondere Chlorid, da es zu Korrosion an Anlagenteilen führen kann.

Zur Wiederverwendung der ionischen Flüssigkeit besteht ein Bedarf an einem einfachen und effektiven Verfahren, Säuren und deren in Salzen vorliegende Anionen, insbesondere kurzkettige Carbonsäuren und deren Anionen sowie unerwünschte anorganische Anionen abzutrennen.

Aus der älteren, nicht vorveröffentlichten europäischen Patentanmeldung mit der Anmeldenummer 11158189.8 (PF 71814) ist ein Verfahren zur Abtrennung von Säuren mit einem schwach basischen Ionentauscher bekannt. Das Verfahren eignet sich nicht zur Abtrennung von Salzen und deren Anionen. Schwach basische Ionenaustauscher sind solche, welche als Ionen-Austausch-Polymer ein Polymer mit primären, sekundären oder tertiären Aminogruppen enthalten und so Säuren binden können. Dabei lagert sich das Säureproton an die Aminogruppe an (Quatemisierung) und das Säureanion ist als Gegenanion gebunden. Anionen von Salzen können daher so nicht abgetrennt werden.

Gewünscht ist daher ein Verfahren, mit dem auch die Anionen von Salzen, insbesondere kurzkettige Anionen von Carbonsäuren abgetrennt werden.

Bei dem Verfahren gemäß Anmeldenummer 11158189.8 (PF 71814) werden alle Säuren abgetrennt, in geringen Mengen auch die Wasserstoffsäure des Anions der ionischen Flüssigkeit, da die Anionen der ionischen Flüssigkeit mit den zugehörigen Wasserstoffsäuren im thermodynamischen Gleichgewicht stehen. Gewünscht ist daher ein Verfahren, bei dem die so abgetrennten Säuren des Anions der ionischen Flüssigkeiten zur Neubildung der ionischen Flüssigkeit verwendet werden können.

Aufgabe der vorliegenden Erfindung ist daher ein einfaches und effektives Verfahren zur Abtrennung von unerwünschten Anionen aus ionischen Flüssigkeiten, bzw. Zusammensetzungen, die ionische Flüssigkeiten enthalten.

Demgemäß wurde das eingangs definierte Verfahren gefunden.

### Zu Verfahrensschritt a)

In einer bevorzugten Ausführungsform haben die Salze K⁺X⁻ einen Schmelzpunkt kleiner 100°C (1 bar, Normaldruck); es handelt sich daher bei den Salzen K⁺X⁻ um ionische Flüssigkeiten.

Insbesondere sind die Salze K⁺X⁻ bei Normalbedingungen (21°C, 1 bar) flüssig.

### Zum Kation K⁺

Geeignete organische Kationen K⁺ sind insbesondere organische Verbindungen mit Heteroatomen, wie Stickstoff, Schwefel, Sauerstoff oder Phosphor.

Insbesondere handelt es sich bei den organische Kationen um Verbindungen mit einer Ammonium-gruppe (Ammonium - Kationen), einer Oxonium -gruppe (Oxonium-Kationen), einer Sulfonium - gruppe (Sulfonium-Kationen) oder einer Phosphonium - Gruppe (Phosphonium-Kationen).

Bevorzugt handelt es sich um ein organisches Kation mit mindestens einem Stickstoffatom.

In einer besonderen Ausführungsform handelt es sich bei den organischen Kationen um Ammonium - Kationen, worunter hier
nicht-aromatische Verbindungen mit lokalisierter positiver Ladung am Stickstoffatom, z.B. Verbindungen mit vierbindigem Stickstoff (quaternäre Ammoniumverbindungen) oder
Verbindungen mit dreibindigem Stickstoff, wobei eine Bindung eine Doppelbindung ist, oder
aromatische Verbindungen mit delokalisierter positiver Ladung und mindestens einem, vorzugsweise ein bis drei Stickstoffatomen im aromatischen Ringsystem
verstanden werden.

Bevorzugte organische Kationen sind quaternäre Ammonium- Kationen, vorzugsweise solche mit drei oder vier aliphatischen Substituenten, besonders bevorzugt C1- bis C12- Alkylgruppen, am Stickstoffatom, welche gegebenenfalls durch Hydroxylgruppen substituiert sein können.

Ebenfalls bevorzugt sind organische Kationen, die ein heterocyclisches Ringsystem enthalten, wobei mindestens ein, vorzugsweise ein bis drei Stickstoffatome, Bestandteil des Ringsystems sind.

In Betracht kommen monocyclische, bicyclische, aromatische oder nicht-aromatische Ringsysteme. Genannt seien z.B. bicyclische Systeme, wie sie in WO 2008/043837 beschrieben sind. Bei den bicyclischen Systemen der WO 2008/043837 handelt es sich um Diazabicyclo- Derivate, vorzugsweise aus einem 7- und einem 6 Ring, welche eine Amidiniumgruppe enthalten; genannt sei insbesondere das 1,8-Diazabicyclo(5.4.0)undec-7-enium- Kation.

Ganz besonders bevorzugte sind Kationen, die ein heterocyclisches Ringsystem mit ein oder zwei Stickstoffatomen als Bestandteil des Ringsystems enthalten.

Als derartige organische Kationen in Betracht kommen z.B. Pyridinium-Kationen, Pyridazinium-Kationen, Pyrimidinium-Kationen, Pyrazinium-Kationen, Imidazolium-Kationen, Pyrazolium-Kationen, Pyrazolinium-Kationen, Imidazolinium-Kationen, Thiazolium-Kationen, Triazolium-Kationen, Pyrrolidinium-Kationen und Imidazolidinium-Kationen. Diese Kationen sind z.B. in WO 2005/113702 aufgeführt. Soweit es für eine positive Ladung am Stickstoffatom oder im aromatischen Ringsystem notwendig ist, sind die Stickstoffatome jeweils durch ein Wasserstoffatom oder eine organische Gruppen mit im Allgemeinen nicht mehr als 20 C-Atomen, vorzugsweise eine Kohlenwasserstoffgruppe, insbesondere eine C1 bis C16 Alkylgruppe, insbesondere eine C1 bis C10, besonders bevorzugt eine C1 bis C4 Alkylgruppen substituiert.

Auch die Kohlenstoffatome des Ringsystems können durch organische Gruppen mit im Allgemeinen nicht mehr als 20 C-Atomen, vorzugsweise eine Kohlenwasserstoffgruppe, insbesondere eine C1 bis C16 Alkylgruppe, insbesondere eine C1 bis C10, besonders bevorzugt eine C1 bis C4 Alkylgruppen substituiert sein.

Besonders bevorzugte Ammonium-Kationen sind quaternäre Ammonium-Kationen, Imidazolium - Kationen, Pyrimidinium- Kationen und Pyrazolium-Kationen.

Besonders bevorzugt handelt es sich bei dem organischen Kation um Imidazolium-Kationen der nachstehenden Formel I, 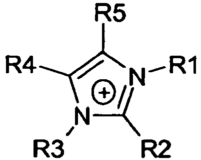 worin
R1 für einen organischen Rest mit 1 bis 20 C-Atomen steht und
R2, R3, R4 und R5 für ein H-Atom oder einen organischen Rest mit 1 bis 20 C-Atomen stehen.

In Formel I stehen R1 und R3 vorzugsweise unabhängig für einen organischen Rest mit 1 bis 10 C-Atomen. Insbesondere stehen R1 und R3 für einen aliphatischen Rest, insbesondere einen aliphatischen Rest ohne weitere Heteroatome, z. B. für eine Alkylgruppe. Besonders bevorzugt stehen R1 und R3 unabhängig voneinander für eine C1- bis C10- bzw. eine C1 bis C4 Alkylgruppe.

In Formel I stehen R2, R4 und R5 vorzugsweise unabhängig für ein H-Atom oder einen organischen Rest mit 1 bis 10 C-Atomen; insbesondere stehen R2, R4 und R5 für ein H-Atom oder einen aliphatischen Rest. Besonders bevorzugt stehen R2, R4 und R5 unabhängig voneinander für ein H-Atom oder eine Alkylgruppe, insbesondere stehen R2, R4 und R5 unabhängig voneinander für ein H-Atom oder eine C1 bis C4 Alkylgruppe. Ganz besonders bevorzugt stehen R2, R4 und R5 jeweils für ein H-Atom.

### Zum Anion X⁻

X⁻ steht für ein organisches Anion mit mindestens 6, insbesondere mindestens 8 C-Atomen. Im Allgemeinen enthält X⁻ nicht mehr als 30, insbesondere nicht mehr als 20 C-Atome.

Insbesondere steht X⁻ für ein Anion mit einer Carboxylat- Sulfonat-, oder Sulfatgruppe.

In einer bevorzugten Ausführungsform enthält X- außer den Sauerstoffatomen und Schwefelatomen in der Carboxylat-, Sulfonat- oder Sulfatgruppe keine weiteren Heteroatome. Insbesondere besteht das Anion X⁻ aus einer anionischen Gruppe ausgewählt aus einer Carboxylat-, einer Sulfonat- oder einer Sulfatgruppe und darüber hinaus aus einem Kohlenwasserstoffrest ohne weitere Heteroatome oder funktionelle Gruppen.

Geeignete Anionen mit einer Sulfatgruppe sind insbesondere Anionen der Formel Rₐ-O-SO₃⁻, wobei Rₐ für eine C6- bis C20 Alkylgruppe steht.

Geeignete Anionen mit einer Sulfonatgruppe sind insbesondere Anionen der Formel R_{b}-SO₃⁻, wobei R_{b} für eine C6- bis C20 Alkylgruppe steht.

Insbesondere steht X⁻ für ein organisches Anion mit einer Carboxylatgruppe.

Als derartige Carboxylate seien insbesondere organische Verbindungen mit 6 bis 20 C-Atomen genannt, die eine Carboxylatgruppe enthalten.

Es kann sich dabei sowohl um aliphatische als auch um aromatische Carboxylate handeln, wobei unter den aromatischen Carboxylate solche verstanden werden, die aromatische Gruppen enthalten. Besonders bevorzugt sind aliphatische oder aromatische Carboxylate, die außer den Sauerstoffatomen der Carboxylatgruppe keine weiteren Heteroatome enthalten, z.B. die Carboxylate der Alkancarbonsäuren, Alkencarbonsäuren, Alkincarbonsäuren, Alkadiencarbonsäuren, Alkatriencarbonsäuren, , Benzoesäure oder Phenylessigsäure. Geeignete Carboxylate der Alkancarbonsäuren, Alkencarbonsäuren und Alkadiencarbonsäuren sind auch als Fettsäure- Carboxylate bekannt.

Besonders bevorzugt handelt es sich bei X⁻ um ein C6- bis C20- Alkanoat.

Ganz besonders bevorzugt sind C6- bis C20- Alkanoate (Carboxylate der Alkancarbonsäuren, insbesondere C8- bis C16- Alkanoate.

Genannt seien insbesondere die Carboxylate der n-Capronsäure (C6-Carbonsäure) n-Caprylsäure (C8-Carbonsäure, Octansäure), n-Caprinsäure (C10-Carbonsäure, Decansäure), Laurinsäure (C12-Carbonsäure, Dodecansäure), Palmitinsäure (C16-Carbonsäure, Hexadecansäure) oder Stearinsäure (C18-Carbonsäure). In einer besonderen Ausführungsform handelt es sich bei den Anionen der Salze um Carboxylate der C6 bis C20 Alkancarbonsäuren, d.h. C6 - C20 Alkanoate. Genannt seien insbesondere C8 bis C16 Alkanoate und in einer besonderen Ausführungsform C8 bis C12 Alkanoate.

Die Wasserstoffsäuren HX der Anionen X⁻ haben keine oder nur eine begrenzte Wasserlöslichkeit. Bei 20°C, 1 bar lösen sich vorzugsweise maximal 100 Gramm HX in 1000 Gramm Wasser, insbesondere lösen sich maximal 50 Gramm HX in 1000 Gramm Wasser, ganz besonders bevorzugt lösen sich maximal 20 Gramm HX in 1000 Gramm Wasser; in einer besonderen Ausführungsform lösen sich maximal 10 Gramm bzw. maximal 5 Gramm HX in Wasser.

Als Salze K⁺ X⁻ seien exemplarisch genannt:
1-Ethyl-3-methyl-imidazolium octanoat,
   1-Methyl-3-methyl-imidazolium octanoat,
   1-Ethyl-3-ethyl-imidazolium octanoat,
1-Ethyl-3-methyl-imidazolium ethylhexanoat,
   1-Methyl-3-methyl-imidazolium ethylhexanoat,
   1-Ethyl-3-ethyl-imidazolium ethylhexanoat,
1-Ethyl-3-methyl-imidazolium isononanoat,
   1-Methyl-3-methyl-imidazolium isononanoat,
   1-Ethyl-3-ethyl-imidazolium isononanoat,
zu weiteren Bestandteilen der unter a) verwendeten Zusammensetzungen.

Die in a) verwendete Zusammensetzung enthält neben den Salzen K⁺X⁻ als Verunreinigungen wasserlösliche Salze (K⁺)ₙ Yⁿ⁻, wobei K⁺ für das obige Kation, Yⁿ⁻ für ein anorganisches Anion oder ein von X⁻ verschiedenes organisches Anion mit maximal 10 C-Atomen und n für 1, 2 oder 3 steht. Insbesondere steht n für 1 oder 2, besonders bevorzugt steht n für 1.

Die wasserlöslichen Salze (K⁺)ₙ Yⁿ⁻ haben vorzugsweise eine Wasserlöslichkeit von mehr als 50 Gramm, insbesondere mehr als 100 Gramm, besonders bevorzugt von mehr als 200 Gramm in 1000 Gramm Wasser (20°C, 1 bar). Soweit es sich bei (K⁺)ₙ Yⁿ⁻ um ionische Flüssigkeiten handelt, sind sie im flüssigen Zustand vorzugsweise in beliebigem Verhältnis mit Wasser mischbar.

Als anorganische Anionen kommen insbesondere Chlorid und Sulfat in Betracht.

Als organische Anionen seinen z. B. solche mit maximal als 8 C- Atome, insbesondere mit maximal 6 C-Atomen bzw. maximal 4 C-Atomen genannt.

Bei den organischen Anionen handelt es sich insbesondere um solche mit mindestens einer Carboxylatgruppe, mindestens einer Sulfonatgruppe oder mindestens einer Sulfatgruppe.

Insbesondere kann es sich um Anionen mit mindestens einer Carboxylatgruppe handeln (kurz Carboxylate).

In einer bevorzugten Ausführungsform handelt es sich bei Carboxylaten Yⁿ⁻ um solche mit einer oder zwei Carboxylatgruppen.

Als Carboxylate Yⁿ⁻ mit einer Carboxylatgruppe seien insbesondere Ameisensäure (C1-Carbon-säure), Essigsäure (C2-Carbonsäure), Propionsäure (C3-Carbonsäure), n-Buttersäure (C4-Carbonsäure) genannt.

Es kann sich bei den Carboxylaten Yⁿ⁻ um aliphatische oder aromatische Carboxylate handeln, wobei unter den aromatischen Carboxylate solche verstanden werden, die aromatische Gruppen enthalten. In Betracht kommen insbesondere auch Carboxylate, welche weitere funktionelle Gruppen, z. B. Hodroxylgruppen, Ethergruppen oder Carbonylgruppen enthalten.

Genannt seinen z. B. Anionen der Glycolsäure, Furandicarbonsäure, Levulinsäure (4-Oxopentansäure) genannt.

Die Zusammensetzung kann verschiedene Salze (K⁺)ₙ Yⁿ⁻ enthalten, insbesondere kann sie verschiedene Salze mit anorganischem Anion Yⁿ⁻ und gleichzeitig verschiedene Salze mit organischem Anion Yⁿ⁻ enthalten.

Der Gehalt aller Salze (K⁺)ₙ Yⁿ⁻ wird in Summe im Allgemeinen nicht höher sein als 200 Gew.-Teile, insbesondere nicht höher sein als 100 Gew. Teile bzw. nicht höher sein als 50 Gew.-Teile (K⁺)ₙ Yⁿ⁻ bezogen auf 100 Gew. teile K⁺X⁻; im Allgemeinen beträgt der Gehalt aller Salze (K⁺)ₙ Yⁿ⁻ in Summe mindestens 1, insbesondere mindestens 5 Gew.- Teile bezogen auf 100 Gew.-Teile K⁺X⁻.

Die wasserlöslichen Salze (K⁺)ₙ Yⁿ⁻ sind Verunreinigungen und können auf unterschiedliche Art in die Zusammensetzung gelangt sein. Sie können durch vorhergehenden Verwendungen der ionischen Flüssigkeit K⁺X⁻ eingebracht oder entstanden sein, letzteres z.B. als Abbauprodukt von Verbindungen sein, mit denen die ionische Flüssigkeit bei einer vorhergehenden Verwendung in Kontakt gekommen ist. Z.B. kann es sich um Abbauprodukte von Cellulose handeln, wenn die ionische Flüssigkeit vorher als Lösemittel für Cellulose verwendet wurde.

Die in a) verwendete Zusammensetzung kann weitere Bestandteile enthalten. Insbesondere kann die Zusammensetzung mit Lösemittel oder weitere Verunreinigungen enthalten, welche durch die vorhergehende Verwendung der ionischen Flüssigkeit eingebracht worden sind. Lösmittel, welche bei einer vorhergehenden Verwendung eingebracht worden sind, sind insbesondere mit der ionischen Flüssigkeit K⁺X⁻ mischbare Lösemittel, z.B. Methanol, Ethanol oder Wasser. Bei der Verwendung von ionischer Flüssigkeit als Lösemittel für Cellulose dient Wasser als Fällungsmittel, um Cellulose in der gewünschten Form, z. B. als Faser, Film oder Perle (beads) auszufällen.

Insbesondere enthält die in a) verwendete Zusammensetzung daher Wasser.

In einer bevorzugten Ausführungsform besteht die in a) verwendete Zusammensetzung zu mehr als 80 Gew. %, besonders bevorzugt zu mehr als 90 Gew. % aus den Salzen K⁺ X⁻ und gegebenenfalls einem damit mischbaren Lösemittel, insbesondere Wasser.

Dabei kann der Anteil von K⁺ X- z.B. 10 bis 95 Gew. %, insbesondere 40 bis 90 Gew. %, oder in einer besonderen Ausführungsform 70 bis 90 Gew. % und der Anteil des Lösemittels (Wasser) entsprechend 5 bis 90 Gew. %, insbesondere 10 bis 60 Gew. % und in einer besonderen Ausführungsform 10 bis 30 Gew. % betragen, wobei die Gewichtsprozente auf die Gewichtssumme von K⁺ X- und das Lösemittel (Wasser) bezogen sind.

Als weitere Verunreinigungen kommen z.B. solche in Betracht, welche sich in der ionischen Flüssigkeit K⁺ X⁻ oder dem Lösemittel lösen oder damit mischbar sind.

Genannt seien z.B. Hemicellulosen; diese werden können bei der Verwendung der ionischen Flüssigkeit als Lösemittel für Cellulose eingebracht werden.

In einer besonderen Ausführungsform enthalten die in a) verwendeten Zusammensetzungen daher auch Hemicellulosen, das sind in Wasser lösliche niedermolekulare Abbauprodukte oder Bestandteile der Cellulose, wie Hexosen, Pentosen und oligomere Hexosen oder Pentosen. Wasserlösliche oligomere Hexosen oder Pentosen haben üblicherweise ein Molekulargewicht kleiner 5000 g/mol.
Der Gehalt der Hemicellulosen in der Zusammensetzung kann z. B. 0,1 bis 5 Gew. Teile, insbesondere 0,5 bis 5 Gew. Teile Hemicellulose auf 100 Gew. teile der Gewichtssumme aus Salzen K⁺ X⁻ und Lösemittel betragen.

### Zu Verfahrensschritt b)

In Verfahrensschritt b) wird der Zusammensetzung eine wasserlösliche Wasserstoffsäure (H⁺)ₘ Z^{m-} zugesetzt. m steht insbesondere für 1 oder 2.

Die wasserlösliche Wasserstoffsäure (H⁺)ₘ Z^{m-} hat vorzugsweise eine Wasserlöslichkeit von mindestens 200 Gramm, insbesondere mindestens 400 Gramm in 1000 Gramm Wasser (20°C, 1 bar); insbesondere ist sie mit Wasser in beliebigen Verhältnissen mischbar.

Vorzugsweise ist der pKs- Wert der Wasserstoffsäure (H⁺)ₘ Z^{m-} kleiner als der pKs Wert von HX, d.h. die Säurestärke von (H⁺)ₘ Z^{m-} ist größer.

Der pks - Wert ist der negative dekadische Logarithmus der Säurekonstanten, KS.
Der pks - Wert wird dazu bei 25°C, 1 bar in Wasser oder Dimethylsulfoxid als Lösemittel gemessen. Es ist daher ausreichend, wenn die Säure entweder in Wasser oder in Dimethylsulfoxid den entsprechenden pKs- Wert hat. Vorzugsweise wird der pKs Wert in Wasser gemessen. Dimethylsulfoxid wird insbesondere dann verwendet, wenn das Anion in Wasser nicht ausreichend löslich ist. Zu beiden Lösemitteln finden sich Literaturangaben in Standardwerken.

Insbesondere ist der pKs Wert der Säure HX um mindestens 0,1, besonders bevorzugt um mindestens 0,5, ganz besonders bevorzugt um mindestens 1 und in einer besonderen Ausführungsform um mindestens 2 größer als der pKs Wert von (H⁺)ₘ Z^{m-}.

Die Wasserstoffsäuren HX haben vorzugsweise einen pKs Wert größer 2, vorzugsweise größer 3 besonders bevorzugt größer 4. Oktansäure hat z. B. einen pKs Wert von 4,8.

Geeignete Wasserstoffsäure (H⁺)ₘ Z^{m-} ist z.B. die Schwefelsäure mit einem pKs Wert von -3 ; für das erfindungsgemäße Verfahren sind jedoch auch Säuren geeignet, deren pKs Wert nur wenig geringer ist als der pKs Wert von HX; so kann z.B. im Falle von Oktansäure als HX als geeignete Säure (H⁺)ₘ Z^{m-} Essigsäure verwendet werden.

Das Anion Z^{m-} der Wasserstoffsäure (H⁺)ₘ Z^{m-} kann mit dem Anion Yⁿ⁻ des wasserlöslichen Salzes (K⁺)ₙ Yⁿ⁻ identisch sein.

Vorzugsweise ist (H⁺)ₘ Z^{m-} ausgewählt aus HCl, HBr, HBF₄, H₃C-COOH, HCOOH, H₃C-O-SO₃H, H₃C-SO₃H, F₃C-O-SO₃H, CH₃-CH₂-COOH, H₂SO₃, H₂SO₄, HNO₃, HClO₄ oder H₃PO₄

(H⁺)ₘ Z^{m-} kann in reiner Form oder vorzugsweise in Form von Lösungen, besonders bevorzugt als wässrige Lösung zugesetzt werden. Insbesondere kann Wasser auch separat zugesetzt werden, z. B. auch in Form von Eis, um gleichzeitig Wärme abzuführen, bzw. aufzunehmen.

(H⁺)ₘ Z^{m-} wird vorzugsweise in solchen Mengen zugesetzt, dass K⁺X⁻ vollständig umgesetzt wird.

Bei der Zugabe kann sowohl die Zusammensetzung als auch das zugesetzte (H⁺)ₘ Z^{m-} bei erhöhter Temperatur vorliegen. Im Allgemeinen ist eine Temperaturerhöhung aber nicht erforderlich und die Zugabe kann bei Raumtemperatur erfolgen.

Durch Zusatz von (H⁺)ₘ Z^{m-} werden die Anionen X⁻ der Salze K⁺X⁻ in die Säuren HX überführt. Die Säuren HX haben nur eine geringe Wasserlöslichkeit, wie bereits oben beschrieben ist; die Säuren HX bilden daher eine organische Phase aus.

Das Kation K⁺ liegt dann entsprechend als wasserlösliches Salz (K⁺)ₘZ^{m-} vor. Auch die wasserlöslichen Salze (K⁺)ₙ Yⁿ⁻ können dabei vollständig oder auch nur zum Teil in die wasserlöslichen Säuren (H⁺)ₙ Yⁿ⁻ überführt werden. Es wird daher eine wässrige Phase erhalten, welche die wasserlösliche Salze (K⁺)ₘZ^{m-} und (K⁺)ₙ Yⁿ⁻ oder Säuren (H⁺)ₙ Yⁿ⁻ enthält.

Durch Zugabe von (H⁺)ₘ Z^{m-} entstehen daher zwei Phasen:
Eine organische Phase, welche HX enthält oder daraus besteht und die vorstehende wässrige Phase.

In einer bevorzugten Ausführungsform wird im Verfahrensschritt b) (H⁺)ₘ Z^{m-} in solchen Mengen zugegeben, dass der pH-Wert der wässrigen Phase kleiner ist als der pKs-Wert von HX, insbesondere um mindestens zwei Einheiten kleiner ist als der pKs-Wert von HX; z. B. bei einem pKs Wert der Oktansäure von 4,8 sollte der pH-Wert der wässrigen Lösung dann höchstens 2,8 sein. HX ist bei derartigen Bedingungen vollständig oder nahezu vollständig in die organische Phase übergegangen.

### Zu Verfahrensschritten c) bis e)

In Verfahrensschritt c) wird die organische Phase abgetrennt.

Die wässrige und organische Phase können leicht getrennt werden, z.B. durch Abdekantieren einer Phase. Gegebenenfalls kann noch ein Extraktionsmittel, z.B. ein organisches Lösemittel zugesetzt werden, welches mit HX mischbar ist. Auf diese Weise können gegebenenfalls Restmengen von HX aus der wässrigen Phase extrahiert werden. Auch wird die Abtrennung erleichtert, weil das Volumen der organischen Phase vergrößert wird.

Das Extraktionsmittel kann auch, wenn gewünscht, bereits in Verfahrensschritt b) gleichzeitig mit der Säure (H⁺)ₘ Z^{m-} und gegebenenfalls Wasser zugesetzt werden.

Als geeignete Extraktionsmittel können z.B. Hexan, tert-Butylmethylether, Essigsäurethylester, Alkohole mit mehr als 5 C-Atomen, wie Heptanol, 2-Ethylhexanol, Toluol, Dichlormethan, Trichlorethan, Benzol, Chlorbenzol, Benzin, Pentanon, Isoamylalkohol, Dichlorethan, Diethylether, Methylisobutylketon, Cyclohexanon, Benzylalkohol, Propylencarbonat, Ethylencarbonat, Dimethylcarbonat oder, Diethylcarbonat verwendet werden.

Die erhaltene wässrige Phase wird über einen stark basischen Anionentauscher geführt (Verfahrensschritt d). Ein stark basischer Anionentauscher bindet Anionen und setzt im Gegenzug OH⁻ frei. So werden die Anionen Z^{m-} und Yⁿ⁻ gebunden und OH- freigesetzt.

Die erhaltene wässrige Lösung enthält daher nur noch das Hydroxyd der Kationen K⁺. Die Protonen H⁺ der Säuren setzen sich mit OH⁻ zu Wasser um.

Übliche stark basische Aniontauscher sind hinreichend bekannt und im Handel erhältlich.

Wenn der stark basische Anionentauscher vollständig mit Anionen beladen ist, muss er regeneriert werden, d.h. die aufgenommenen Anionen werden durch Waschen des Aniontauschers mit einer OH⁻ enthaltenden Lösung wieder entfernt und der Anionentauscher wird wieder in die Hydroxyd-form überführt.

Im abschließenden Verfahrensschritt e) wird die organische Phase (enthaltend HX) und das erhaltene wässrige Gemisch (enthaltend K⁺ OH⁻), gegebenenfalls unter Zusatz von weiterem HX, zu K⁺X⁻ und H₂O umgesetzt. Im Allgemeinen tritt die Umsetzung von K⁺OH⁻ mit HX zu K⁺X⁻ + H₂O bei Raumtemperatur sofort ein; eine Temperaturerhöhung ist nicht notwendig.

Ein Zusatz von weiterem HX in Verfahrensschritt e) kommt insbesondere in Betracht weil das Kation K⁺ vorher mit Anionen X⁻ und Yⁿ⁻ vorlag; da Yⁿ⁻ aber entfernt wurde, wird ggf. weiteres HX für eine vollständige Umsetzung aller K⁺ benötigt.

Wenn in den vorhergehenden Verfahrensschritten kein organisches Extraktionsmittel verwendet wurde, wird nur noch eine wässrige Phase erhalten. Bei vorheriger Mitverwendung eines Extraktionsmittels wird naturgemäß weiterhin eine organische Phase erhalten, welche aber im Wesentlichen nur noch aus dem Extraktionsmittel besteht. Die wässrige Phase kann dann wiederum leicht abgetrennt werden (siehe oben).

Bevorzugt wird HX (aus der organischen Phase und ggf. zusätzliches HX) in solchen Mengen zugesetzt, dass der pH-Wert der erhaltenen wässrigen Phase größer ist als der pKs-Wert von HX; besonders bevorzugt ist der pH-Wert der erhaltenen wässrigen Phase mindestens zwei Einheiten größer als der pKs-Wert von HX, z. B. bei einem pKs Wert der Oktansäure von 4,8 sollte der pH-Wert der erhaltenen wässrigen Phase dann mindestens 6,8 sein.

Wasser kann aus der wässrigen Phase, wenn für die weitere Verwendung gewünscht oder notwendig, durch einfache Verfahren abgetrennt werden, z.B. durch Destillation.

### Weitere Verfahrensschritte

Gegebenenfalls können vor oder nach der Durchführung des erfindungsgemäßen Verfahrens weitere Maßnahmen zur Reinigung von K⁺X⁻ vorgenommen werden.

Die Zusammensetzungen unter a) können z. B. auch unerwünschte Kationen enthalten, welche durch eine vorhergehende Verwendung der ionischen Flüssigkeit K⁺X⁻ eingebracht worden sind. Es kann sich dabei z. B. um Metallkationen, wie Natrium- oder Kaliumionen, oder um chemisch modifizierte Kationen K⁺ handeln. Im Falle von Imidazoliumkationen K⁺ kann es z.B. bei verschiedenen Verwendungen zu Additions- oder Substitutionsreaktionen an das C-Atom zwischen den beiden Stickstoffatomen (R2 Position in Formel I) kommen. Im Falle der Verwendung von Imidazoliumverbindungen als Lösemittel für Cellulose können sich Abbauprodukte wie Formaldehyd an das R2-Kohlenstoffatom addieren (R2 in Formel I ist dann ein Methylol-rest).

Derartige unerwünschte Kationen können z.B. durch die in WO 2009/027250 beschriebene Molekulardestillation ionischer Flüssigkeiten abgetrennt werden, vorzugsweise im Anschluss an das hier beschriebene Verfahren.

Die Zusammensetzungen unter a) können z.B. auch unerwünschte Säuren enthalten, welche ebenfalls durch eine vorhergehende Verwendung der ionischen Flüssigkeit K⁺X⁻ eingebracht wurden oder entstanden sind.

Derartige Säuren können z.B. vor Durchführung des hier beschriebenen Verfahrens abgetrennt werden. Bei den Säuren kann es sich insbesondere auch um Säuren HX handeln, welche durch die vorhergehende Verwendung der ionischen Flüssigkeit aus dem Anion X⁻ entstanden sind. Ein Verfahren zur Abtrennung derartiger Säuren ist in der Patentanmeldung EP Aktenzeichen 11158189.8 (PF 71814) beschrieben. Demnach können derartige Säuren, insbesondere auch HX, durch einen schwach basischen Anionentauscher entfernt werden. Schwach basische Ionenaustauscher sind solche, welche als Ionen-Austausch-Polymer ein Polymer mit primären, sekundären oder tertiären Aminogruppen enthalten und so Säuren binden können. Dabei lagert sich das Säureproton an die Aminogruppe an (Quatemisierung) und das Säureanion ist als Gegenanion gebunden.

Vorab abgetrennte Säuren HX können bei dem erfindungsgemäßen Verfahren wiederverwendet werden. Dazu muss das im schwach basischen Ionentauscher gebunden HX wieder gewonnen werden. Vorteilhafterweise wird der mit HX beladene, schwach basische Anionentauscher zunächst mit einer Base, z.B. NaOH, gespült; dadurch entsteht zunächst das entsprechende Salz der Base, z.B. NaX. Das Salz (NaX) kann durch Zugabe einer Säure und gegebenenfalls von organischem Lösemittel leicht in HX überführt werden. HX, gegebenenfalls auch zusammen mit organischem Lösemittel, kann dann in Verfahrensschritt e) als weiteres HX zugesetzt werden (siehe oben).

Das erfindungsgemäße Verfahren ist ein einfaches und sehr effektives Verfahren zur Reinigung von Salzen K⁺X⁻, wobei K⁺X⁻ insbesondere ionischen Flüssigkeiten sind.

### Beispiel

### Ausgangsstoffe

Ionische Flüssigkeit (K⁺ X⁻):
1-Ethyl-3-ethyl-imidazolium octanoat (kurz EEIM-Oct)

Als Zusammensetzung wurde eine Zusammensetzung verwendet, welche wiederholt zum Auflösen von Cellulose eingesetzt wurde. Diese bestand aus EEIM-Oct und enthielt durch die wiederholte Verwendung Wasser und eine Reihe von Verunreinigungen.

Die Zusammensetzung enthielt 24,9 Gew. % aus EEIM und 25,8% Gew. % Octanoat.

Der Wassergehalt in der Zusammensetzung betrug 36,7 Gew. %

Der Gehalt an weiteren Anionen Yⁿ⁻ in der Zusammensetzung vor Durchführung und nach Durchführung des erfindungsgemäßen Verfahrens ist in der Tabelle angegeben.

Der Gehalt der Säureanionen gemäß Tabelle I wurde vor und nach Durchführung des erfindungsgemäßen Verfahrens durch Kapillarelektrophorese bestimmt.

In der Kapillarelektrophorese werden die verschiedenen Säureanionen anhand der unterschiedlichen Wanderungsgeschwindigkeiten im elektrischen Feld getrennt. Zur Detektion wird der Lösung Benzoesäure dazugegeben, deren UV-Absorption bei 225 nm liegt.

### Durchführung

### Verfahrensschritte a) bis c)

4615,4 g der Zusammensetzung wurden mit 571 g einer 95% Schwefelsäure umgesetzt, wobei aus EEIM-Oct EEIM Sulfat gemäß folgender Reaktionsgleichung entsteht. Es wird eine wässrige Phase, enthaltend EEIM-Sulfat und die Verunreinigungen EEIM-Y, und Octansäure als organische Phase erhalten.

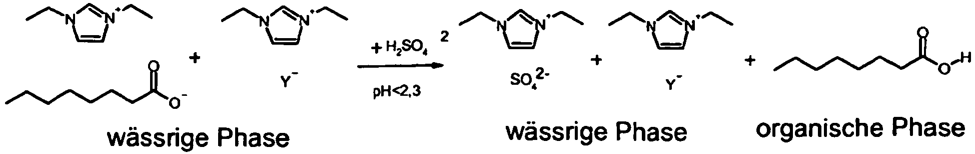

Die Octansäure wurde dreimal mit je 2,5 L Heptan extrahiert.

### Verfahrensschritt d)

Danach wurde die wässrige Phase (4,895 kg) über einen stark basischen Ionentauscher (19,2 kg; Kapazität 1,15 mol/kg) geführt, wobei das EEIM-Sulfat und die Verunreinigungen EEIM-Y in das EEIM-Hydroxyd überführt wurde:

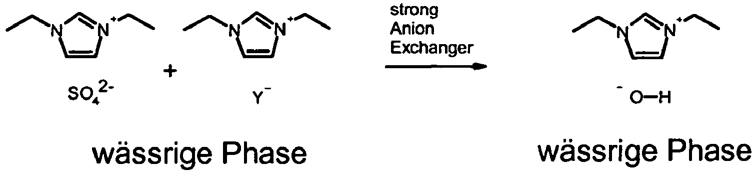

### Verfahrensschritt e)

Im letzten Schritt wurden 25 kg Eluat aus dem Ionentauscher (EEIM-Hydroxyd) mit der organischen Phase aus c) (Octansäure) und mit 460 g frischer Octansäure wieder zu EEIM-Oct umgesetzt.

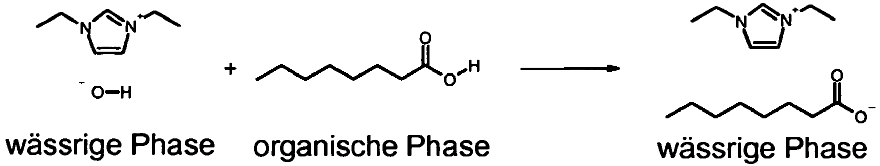

Nach Destillation des Wassers erhielt man 2,829 kg EEIM Octanoat mit einem Wassergehalt von 5%.

**Tabelle:**

| Gehalt an Anionen Yⁿ⁻: | | | | | |
|---|---|---|---|---|---|
| | Vorher | | Nachher | | nacher/vorher |
| Anion | Gew. % in Lösung | Massenverhältnis Octanoat zu Anion in % | Gew.% in Lösung | Massenverhältnis Octanoat zu Anion in % | Abreicherung auf % des Ausgangswertes |
| Octanoat | 24,850 | 100,000 | 47,870 | 100,000 | 100 |
| Formiat | 1,110 | 4,467 | 0,140 | 0,292 | 7 |
| Adipat | 0,160 | 0,644 | 0,006 | 0,013 | 2 |
| der Glutarsäure | 0,080 | 0,322 | 0,003 | 0,006 | 2 |
| der Glykolsäure | 0,110 | 0,443 | 0,040 | 0,084 | 19 |
| Acetat | 0,190 | 0,765 | 0,080 | 0,167 | 22 |
| Lactat | 0,020 | 0,080 | 0,009 | 0,019 | 23 |
| Propionat | 0,080 | 0,322 | 0,040 | 0,084 | 26 |
| der Lävulinsäure | 0,020 | 0,080 | 0,010 | 0,021 | 26 |
| Chlorid | 0,039 | 0,157 | 0,001 | 0,002 | 1 |
| Sulfat | 0,028 | 0,113 | 0,001 | 0,002 | 2 |

## Patentansprüche

1. Verfahren zur Reinigung von Salzen K⁺X⁻, wobei K⁺ für ein beliebiges Kation und X⁻ für ein organisches Anion mit mindestens 6 C-Atomen steht, **dadurch gekennzeichnet, dass**
a) die Salze K⁺X⁻ in Zusammensetzungen vorliegen, die als Verunreinigungen wasserlösliche Salze (K⁺)ₙ Yⁿ⁻ enthalten, wobei K⁺ für das obige Kation, Yⁿ⁻ für ein anorganisches Anion oder ein von X⁻ verschiedenes organisches Anion mit maximal 10 C-Atomen und n für 1, 2 oder 3 steht,
b) die Anionen X⁻ der Salze K⁺X⁻ durch Zusatz einer wasserlöslichen Wasserstoffsäure (H⁺)ₘ Z^{m-}, wobei m für 1, 2 oder 3 steht, in die Säuren HX überführt werden, wobei sich zwei Phasen ausbilden, eine organische Phase, welche HX enthält, und eine wässrige Phase, welche die wasserlösliche Salze (K⁺)ₘZ^{m-} und (K⁺)ₙ Yⁿ⁻ oder Säuren (H⁺)ₙ Yⁿ⁻ enthält,
c) die organische Phase abgetrennt wird,
d) die wässrige Phase über einen stark basischen Anionentauscher geführt wird, welcher die Anionen Z^{m-} und Yⁿ⁻ bindet und OH⁻ frei setzt, so dass ein wässriges Gemisch, enthaltend K⁺ OH⁻ erhalten wird und
e) die organische Phase (enthaltend HX) und das erhaltene wässrige Gemisch (enthaltend KOH), gegebenenfalls unter Zusatz von weiterem HX, zu K⁺X⁻ und H₂O umgesetzt werden.

2. Verfahren gemäß Anspruch 1, **dadurch gekennzeichnet, dass** das Salz K⁺X⁻ einen Schmelzpunkt kleiner 100°C (1 bar, Normaldruck) hat.

3. Verfahren gemäß Anspruch 1 oder 2, **dadurch gekennzeichnet, dass** das Salz K⁺X⁻ bei 21°C, 1 bar (Normalbedingungen) flüssig ist.

4. Verfahren gemäß einem der Ansprüche 1 bis 3, **dadurch gekennzeichnet, dass** es sich bei K⁺ um ein organisches Kation mit einem heterocyclischen Ringsystem und mindestens einem Stickstoffatom als Bestandteil des Ringsystems handelt.

5. Verfahren gemäß einem der Ansprüche 1 bis 4, **dadurch gekennzeichnet, dass** es sich bei K⁺ um ein Imidazolium-Kation der nachstehenden Formel I handelt, 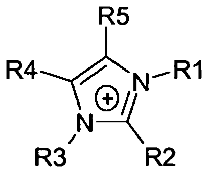 worin
R1 für einen organischen Rest mit 1 bis 20 C-Atomen steht und
R2, R3, R4 und R5 für ein H-Atom oder einen organischen Rest mit 1 bis 20 C-Atomen stehen.

6. Verfahren gemäß einem der Ansprüche 1 bis 5, **dadurch gekennzeichnet, dass** X⁻ für ein organisches Anion mit einer Carboxylatgruppe steht.

7. Verfahren gemäß einem der Ansprüche 1 bis 6, **dadurch gekennzeichnet, dass** es sich bei X- um ein C6- bis C20- Alkanoat handelt.

8. Verfahren gemäß einem der Ansprüche 1 bis 7, **dadurch gekennzeichnet, dass** die Zusammensetzungen zu mehr als 80 Gew. % aus den Salzen K⁺ X⁻ und gegebenenfalls einem damit mischbaren Lösemittel besteht.

9. Verfahren gemäß Anspruch 8, **dadurch gekennzeichnet, dass** es sich bei dem mit K⁺ X⁻ mischbarem Lösemittel um Wasser handelt.

10. Verfahren gemäß einem der Ansprüche 1 bis 9, **dadurch gekennzeichnet, dass** die Zusammensetzungen 0,1 bis 5 Gew. teile Hemicellulose auf 100 Gew. teile der Gewichtssumme aus Salzen K⁺ X⁻ und Lösemittel enthalten.

11. Verfahren gemäß einem der Ansprüche 1 bis 10, **dadurch gekennzeichnet, dass** es sich bei Yⁿ⁻ um ein organisches Anion mit einer oder zwei Carboxylatgruppen oder ein anorganisches Anion, ausgewählt aus Chlorid oder Sulfat handelt.

12. Verfahren gemäß einem der Ansprüche 1 bis 11, **dadurch gekennzeichnet, dass** (H⁺)ₘ Z^{m-} ausgewählt ist aus HCl, HBr, HBF₄, H₃C-COOH, HCOOH, H₃C-O-SO₃H, H₃C-SO₃H, F₃C-O-SO₃H, CH₃-CH₂-COOH, H₂SO₃, H₂SO₄, HNO₃, HClO₄ oder H₃PO₄.

13. Verfahren gemäß einem der Ansprüche 1 bis 12, **dadurch gekennzeichnet, dass** zur Abtrennung der organischen Phase in Schritt c) zusätzlich ein Extraktionsmittel zugesetzt wird.

14. Verfahren gemäß Anspruch 13, **dadurch gekennzeichnet, dass** das Extraktionsmittel ausgewählt ist aus Hexan, tert-Butylmethylether, Essigsäurethylester, Alkohole mit mehr als 5 C-Atomen, wie Heptanol, 2-Ethylhexanol, Toluol, Dichlormethan, Trichlorethan, Benzol, Chlorbenzol, Benzin, Pentanon, Isoamylalkohol, Dichlorethan, Diethylether, Methylisobutylketon, Cyclohexanon, Benzylalkohol, Propylencarbonat, Ethylencarbonat, Dimethylcarbonat, Diethylcarbonat.

15. Verfahren gemäß einem der Ansprüche 1 bis 14, **dadurch gekennzeichnet, dass**
- die Zusammensetzungen unter a) bereits Säuren HX als Verunreinigungen enthalten,
- HX vorab durch schwach basischen Ionentauscher abgetrennt wird und
- das vorab abgetrennte HX in Verfahrensschritt e) zugesetzt wird.

## Claims

1. A process for purifying salts Cat⁺X⁻, where Cat⁺ is any cation and X⁻ is an organic anion having at least 6 carbon atoms, wherein
a) the salts Cat⁺X⁻ are present in compositions comprising water-soluble salts (Cat⁺)ₙYⁿ⁻, where Cat⁺ is the above cation, Yⁿ⁻ is an inorganic anion or an organic anion which is different from X⁻ and has not more than 10 carbon atoms and n is 1, 2 or 3 as impurities,
b) the anions X⁻ of the salts Cat⁺X⁻ are converted by addition of a water-soluble protic acid (H⁺)ₘZ^{m-}, where m is 1, 2 or 3, into the acids HX, forming two phases, namely an organic phase comprising HX and an aqueous phase comprising the water-soluble salts (Cat⁺)ₘZ^{m-} and (Cat⁺)ₙYⁿ⁻ or acids (H⁺)ₙYⁿ⁻,
c) the organic phase is separated off,
d) the aqueous phase is passed over a strongly basic anion exchanger which binds the anions Z^{m-} and Yⁿ⁻ and liberates OH ⁻ so as to give an aqueous mixture comprising Cat⁺ OH⁻ and
e) the organic phase (comprising HX) and the aqueous mixture obtained (comprising CatH) are, optionally with addition of further HX, converted into Cat⁺X⁻ and H₂O.

2. The process according to claim 1, wherein the salt Cat⁺X⁻ has a melting point of less than 100°C (1 bar, atmospheric pressure).

3. The process according to claim 1 or 2, wherein the salt Cat⁺X⁻ is liquid at 21°C, 1 bar (standard conditions).

4. The process according to any of claims 1 to 3, wherein Cat⁺ is an organic cation having a heterocyclic ring system and at least one nitrogen atom as constituent of the ring system.

5. The process according to any of claims 1 to 4, wherein Cat⁺ is an imidazolium cation of the formula I below, 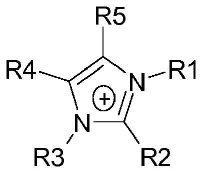 where
R1 is an organic radical having from 1 to 20 carbon atoms and
R2, R3, R4 and R5 are each an H atom or an organic radical having from 1 to 20 carbon atoms.

6. The process according to any of claims 1 to 5, wherein X⁻ is an organic anion having a carboxylate group.

7. The process according to any of claims 1 to 6, wherein X⁻ is a C6-C20-alkanoate.

8. The process according to any of claims 1 to 7, wherein the compositions comprise more than 80% by weight of the salts Cat⁺X⁻ and optionally a solvent miscible therewith.

9. The process according to claim 8, wherein the solvent miscible with Cat⁺X⁻ is water.

10. The process according to any of claims 1 to 9, wherein the compositions comprise from 0.1 to 5 parts by weight of hemicellulose per 100 parts by weight of the total weight of salts Cat⁺X⁻ and solvent.

11. The process according to any of claims 1 to 10, wherein Yⁿ⁻ is an organic anion having one or two carboxylate groups or an inorganic anion selected from among chloride and sulfate.

12. The process according to any of claims 1 to 11, wherein (H⁺)ₘ Z^{m-} is selected from among HCl, HBr, HBF₄, H₃C-COOH, HCOOH, H₃C-O-SO₃H, H₃C-SO₃H, F₃C-O-SO₃H, CH₃-CH₂-COOH, H₂SO₃, H₂SO₄, HNO₃, HClO₄ and H₃PO₄.

13. The process according to any of claims 1 to 12, wherein an extractant is additionally added for separating off the organic phase in step c).

14. The process according to claim 13, wherein the extractant is selected from among hexane, tert-butyl methyl ether, ethyl acetate, alcohols having more than 5 carbon atoms, e.g. heptanol, 2-ethylhexanol, toluene, dichloromethane, trichloroethane, benzene, chlorobenzene, petroleum spirit, pentanone, isoamyl alcohol, dichloroethane, diethyl ether, methyl isobutyl ketone, cyclohexanone, benzyl alcohol, propylene carbonate, ethylene carbonate, dimethyl carbonate, diethyl carbonate.

15. The process according to any of claims 1 to 14, wherein
- the compositions under a) comprise acids HX as impurities,
- HX is separated off beforehand by means of weakly basic ion exchangers and
- the HX which has been separated off beforehand is added in process step e).

## Revendications

1. Procédé pour la purification de sels K⁺X⁻, K⁺ représentant un cation quelconque et X⁻ représentant un anion organique comprenant au moins 6 atomes de carbone, **caractérisé en ce que**
a) les sels K⁺X⁻ se trouvent dans des compositions qui contiennent, comme impuretés, des sels solubles dans l'eau (K⁺)ₙYⁿ⁻, K⁺ représentant le cation ci-dessus, Yⁿ⁻ représentant un anion inorganique ou un anion organique différent de X⁻ comprenant au maximum 10 atomes de carbone et n valant 1, 2 ou 3,
b) les anions X⁻ des sels K⁺X⁻ sont transformés, par addition d'un hydracide soluble dans l'eau (H⁺)ₘZ^{m-}, m valant 1, 2 ou 3, en acides HX, avec formation de deux phases, une phase organique qui contient HX et une phase aqueuse qui contient les sels solubles dans l'eau (K⁺)ₘZ^{m-} et (K⁺)ₙYⁿ⁻ ou les acides (H⁺)ₙYⁿ⁻,
c) la phase organique est séparée,
d) la phase aqueuse est guidée sur un échangeur anionique fortement basique, qui lie les anions Z^{m-} et Yⁿ⁻ et qui libère OH⁻, de telle sorte qu'on obtient un mélange aqueux contenant K⁺OH⁻ et
e) la phase organique (contenant HX) et le mélange aqueux obtenu (contenant KOH) sont transformés, le cas échéant avec addition de HX supplémentaire, en K⁺X⁻ et H₂O.

2. Procédé selon la revendication 1, **caractérisé en ce que** le sel K⁺X⁻ présente un point de fusion inférieur à 100°C (1 bar, pression normale).

3. Procédé selon la revendication 1 ou 2, **caractérisé en ce que** le sel K⁺X⁻ est liquide à 21°C, 1 bar (conditions normales).

4. Procédé selon l'une quelconque des revendications 1 à 3, **caractérisé en ce qu'**il s'agit, pour K⁺, d'un cation organique présentant un système cyclique hétérocyclique et au moins un atome d'azote comme constituant du système cyclique.

5. Procédé selon l'une quelconque des revendications 1 à 4, **caractérisé en ce qu'**il s'agit, pour K⁺, d'un cation d'imidazolium de la formule I suivante, 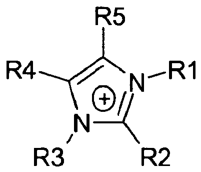 dans laquelle
R1 représente un radical organique comprenant 1 à 20 atomes de carbone et
R2, R3, R4 et R5 représentent un atome d'hydrogène ou un radical organique comprenant 1 à 20 atomes de carbone.

6. Procédé selon l'une quelconque des revendications 1 à 5, **caractérisé en ce que** X⁻ représente un anion organique présentant un groupe carboxylate.

7. Procédé selon l'une quelconque des revendications 1 à 6, **caractérisé en ce qu'**il s'agit, pour X⁻, d'un alcanoate en C6 à C20.

8. Procédé selon l'une quelconque des revendications 1 à 7, **caractérisé en ce que** les compositions sont constituées, à raison de plus de 80% en poids, des sels K⁺X⁻ et le cas échéant d'un solvant miscible avec ceux-ci.

9. Procédé selon la revendication 8, **caractérisé en ce qu'**il s'agit, pour le solvant miscible avec K⁺X⁻, d'eau.

10. Procédé selon l'une quelconque des revendications 1 à 9, **caractérisé en ce que** des compositions contiennent 0,1 à 5 parties en poids d'hémicellulose pour 100 parties en poids de la somme pondérale des sels K⁺X⁻ et du solvant.

11. Procédé selon l'une quelconque des revendications 1 à 10, **caractérisé en ce qu'**il s'agit, pour Yⁿ⁻, d'un anion organique comprenant un ou deux groupes carboxylate ou d'un anion inorganique choisi parmi le chlorure ou le sulfate.

12. Procédé selon l'une quelconque des revendications 1 à 11, **caractérisé en ce que** (H⁺)ₘZ^{m-} est choisi parmi HCl, HBr, HBF₄, H₃C-COOH, HCOOH, H₃C-O-SO₃H, H₃C-SO₃H, F₃C-O-SO₃H, CH₃-CH₂-COOH, H₂SO₃, H₂SO₄, HNO₃, HClO₄ ou H₃PO₄.

13. Procédé selon l'une quelconque des revendications 1 à 12, **caractérisé en ce qu'**un agent d'extraction est ajouté en plus dans l'étape c) pour la séparation de la phase organique.

14. Procédé selon la revendication 13, **caractérisé en ce que** l'agent d'extraction est choisi parmi l'hexane, le tert-butylméthyléther, l'ester éthylique de l'acide acétique, les alcools comprenant plus de 5 atomes de carbone, tels que l'heptanol, le 2-éthylhexanol, le toluène, le dichlorométhane, le trichloroéthane, le benzène, le chlorobenzène, la benzine, la pentanone, l'alcool isoamylique, le dichloroéthane, le diéthyléther, la méthylisobutylcétone, la cyclohexanone, l'alcool benzylique, le carbonate de propylène, le carbonate d'éthylène, le carbonate de diméthyle, le carbonate de diéthyle.

15. Procédé selon l'une quelconque des revendications 1 à 14, **caractérisé en ce que**
- les compositions au point a) contiennent déjà des acides HX comme impuretés,
- HX est séparé au préalable par des échangeurs ioniques faiblement basiques et
- le HX séparé au préalable est ajouté dans l'étape de procédé e).
